Europäisches Patentamt

European Patent Office

Office européen des brevets

(19)

(11) Publication number: **0 453 569 B1**

# EUROPEAN PATENT SPECIFICATION

(12)

(45) Date of publication of patent specification: **06.09.95**  (51) Int. Cl.⁶: **C07C  29/84**

(21) Application number: **90909186.0**

(22) Date of filing: **18.06.90**

(86) International application number:
**PCT/JP90/00793**

(87) International publication number:
**WO 91/07367 (30.05.91 91/12)**

(54) **MANUFACTURE OF ABSOLUTE ALCOHOL.**

(30) Priority: **14.11.89 JP 294032/89**

(43) Date of publication of application:
**30.10.91 Bulletin  91/44**

(45) Publication of the grant of the patent:
**06.09.95 Bulletin  95/36**

(84) Designated Contracting States:
**DE FR GB**

(56) References cited:
**AU-A- 8 813 867**
**JP-A- 5 461 107**
**JP-A- 6 430 592**
**JP-A- 6 470 421**

**No relevant documents disclosed**

(73) Proprietor: **JAPAN as represented by MINISTRY OF INTERNATIONAL TRADE AND INDUSTRY, Director General of Basic Industries Bureau**
**1-1, Kasumigaseki 1-chome,**
**Chiyoda-ku**

**Tokyo 100 (JP)**

(72) Inventor: **KANO, Yoshikazu, c/o Mitsubishi Jukogyo K.K.**
**Yokohama seisakusho,**
**12, Nishiki-cho,**
**Naka-ku**
**Yokohama-shi,**
**Kanagawa 231 (JP)**
Inventor: **HORIZOE, Hirotoshi, Mitsubishi Jukogyo K.K.**
**Hiroshima Kenkyusho,**
**6-22, Kanonshinmachi 4-chome**
**Nishi-ku,**
**Hiroshima-shi,**
**Hiroshima 733 (JP)**
Inventor: **TANIMOTO, Tetsuya, Mitsubishi Jukogyo K.K.**
**Hiroshima Kenkyusho,**
**6-22 Kanonshinmachi 4-chome**
**Nishi-ku,**
**Hiroshima-shi,**
**Hiroshima 733 (JP)**

Inventor: **YAMAMOTO, Itsuo, Mitsubishi Jukogyo K.K.**
**Hiroshima Kenkyusho,**
**6-22, Kanonshinmachi 4-chome**
**Nishi-ku,**
**Hiroshima-shi, Hiroshima 733 (JP)**

(74) Representative: **Baverstock, Michael George Douglas et al**
**BOULT, WADE & TENNANT**
**27 Furnival Street**
**London, EC4A 1PO (GB)**

## Description

Technical Field of Invention

This invention relates to a process for concentrating and purifying alcohol and more particularly, it is concerned with a process suitable for obtaining high purity alcohol with energy saving by concentrating and purifying synthetic alcohols, waste aqueous alcohol solutions in the food industry, fermented alcohols, etc.

Technical Background

Fermented alcohols from carbohydrates such as sweet potatoes, corns, etc. are important raw materials for drinking or industrial use, but an aqueous alcohol solution obtained by the fermentation method has a low alcohol concentration, i.e. 10 to 20 % by weight and accordingly, it is required to concentrate it to about 95 to 100 % by weight.

Up to the present time, a distillation method has been employed as the concentrating method, but this distillation method is not economical because of difficulty in recovering the evaporation latent heat of alcohol and water, as a predominant component. Thus, it has eagerly been desired to develop a concentrating method of energy saving type.

In the conventional distillation method, an energy corresponding to about 3000 kcal/kg • alcohol is required for concentrating alcohol from 10 % by weight to 95 % by weight. For concentrating alcohol from 95 % by weight to absolute alcohol of at least 99.2 % by weight, azeotropic distillation using diethyl ether, benzene or cyclohexane has been carried out with about 1000 to 2000 kcal/kg • alcohol. Thus, energy saving is also desired.

On the other hand, there has been proposed a method comprising extracting and separating alcohol from water by the use of carbon dioxide under supercritical state or pseudocritical state, thereby concentrating the alcohol, as a concentrating method of energy saving type (Japanese Patent Laid-Open Publication Nos. 56201/1981 and 141528/1984).

In the case of using carbon dioxide as a solvent, however, it has lately been reported that the solubility of alcohol is so low that a large amount or carbon dioxide is required, the selective extraction of alcohol is limited and the maximum alcohol concentration is limited to about 91 % by weight, and accordingly, it is impossible to obtain a concentration exceeding this limit.

EP-A-1 306 358 describes a process for removing water from an aqueous mixture of alcohols comprising ethanol and ethanol by a distillation procedure involving adding a $C_2$-$C_6$ hydrocarbon.

Disclosure of the Invention

The present invention aims at providing a process for concentrating alcohol to a degree of concentration of 99 % by weight or more with much less energy as compared with the distillation method of the prior art.

That is, the inventors have made various studies on a process for producing absolute alcohol with a purity of at least 99 % by weight, being valuable on a alcohol market with energy saving and consequently, have found that absolute alcohol can readily be obtained by adding a hydrocarbon solvent consisting of propane, propylene, n-butane, i-butane or mixtures thereof, as an extracting solvent, to an aqueous alcohol solution as a raw material, and then subjecting the mixture to pressurized extractive distillation and/or supplying the most part of the heat source of the pressurized extractive distillation column by the compressive heat generated during recompressing and recycling the overhead gas of the pressurized extractive distillation column, whereby the energy can more be saved as compared with the prior art distillation method. The present invention is based on this finding.

That is, the present invention provides a process for the production of absolute alcohol, which comprises feeding a raw material containing ethanol and water, as predominant components, to the middle part of a first distillation column, feeding a hydrocarbon solvent selected from hydrocarbons of $C_3$-$C_4$ to the upper part of the first distilling column, maintaining the hydrocarbons at such a temperature and pressure that the liquid and gas of the hydrocarbon are simultaneously present in the first distilling column, withdrawing ethanol and liquid hydrocarbons, substantially free from water content, from the lower part of the first distillation column and withdrawing water and vapor hydrocarbons, substantially free from ethanol, from the upper part of the distillation column, characterized in that when the mixed solution of absolute alcohol and hydrocarbons at the lower part of the first distillation column is fed to a second distillation column, where the hydrocarbon solvent is stripped, the gaseous phase at the upper part of the second

3

distillation column is mixed with the gaseous phase at the upper part of the first distillation column and pressed, after using the compression heat thereof as a heat source of the first distillation column, the mixture is cooled and liquefied, and after the water content is separated, it is recycled to the upper parts of the second distillation column and the first distillation column.

Brief Description of the Drawings

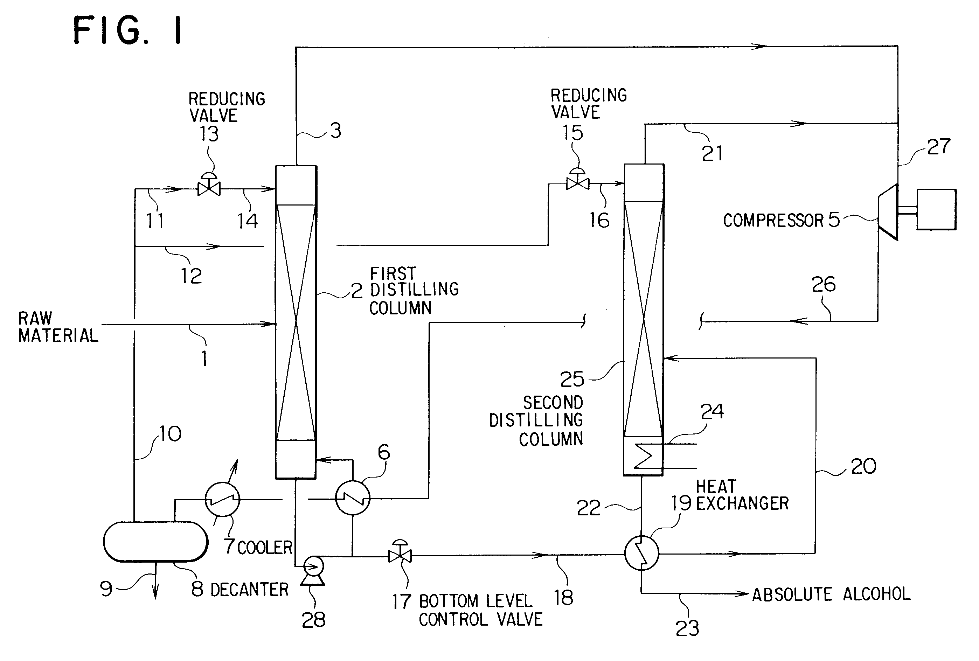

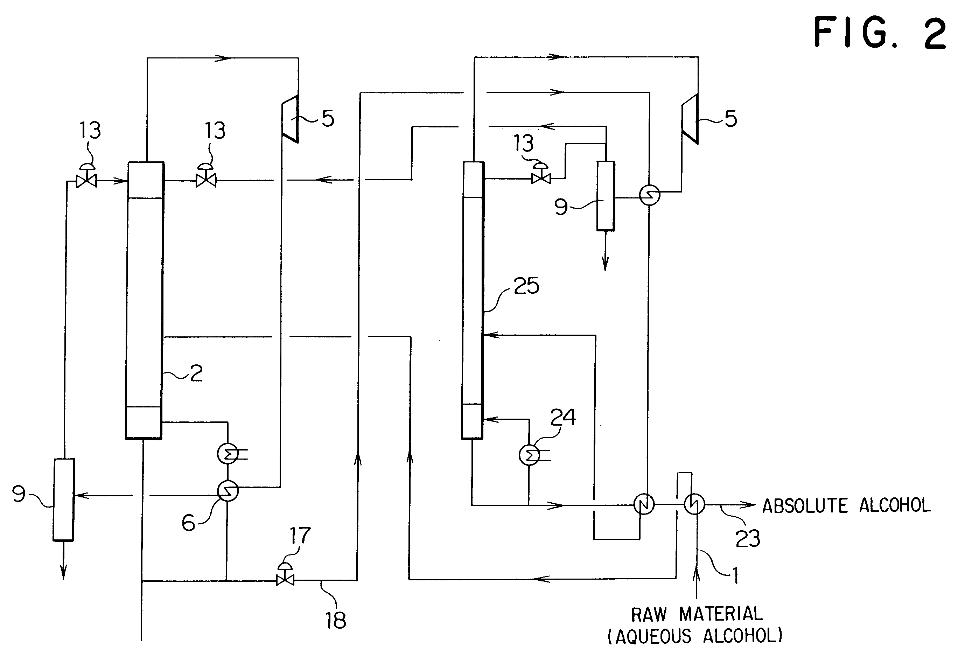

Fig. 1 is a process flow for carrying out the present invention and Fig. 2 is a process flow before the improvement according to the present invention.

Best Embodiment for carrying out the Present Invention

One embodiment of the present invention will now be illustrated in detail by Fig. 1 to clarify the actions thereof.

Referring to Fig. 1, an aqueous alcohol solution, as a raw material, from a feed line 1 is fed to the middle part of a first distillation column 2 and a hydrocarbon solvent is fed to the upper part of the first distillation column 2 from a line 14. When the aqueous alcohol solution is allowed to be present under such a condition that the gas and liquid of the hydrocarbons coexist, the alcohol is selectively extracted by the liquid hydrocarbons. Under such a condition that the liquid hydrocarbon are present in a relatively larger amount than the alcohol, water content is hardly dissolved in a mixed liquid solution of the hydrocarbon and alcohol and the water content can be selectively transferred to the hydrocarbon gas phase side by providing such a condition that the water concentration in the hydrocarbon gas phase be less than the saturated concentration of water. As the hydrocarbon, it is preferable to use propane, propylene, n-butane or i-butane in view of the interaction of alcohol or water with the hydrocarbon. Thus, the alcohol and water can be separated to obtain absolute alcohol. For the purpose of obtaining the provision that both the gas and liquid of the hydrocarbon coexist in the first distillation column 2 for effecting the pressurized extraction, it is required to adjust the temperature to at most about the critical temperature Tc of the hydrocarbon and the pressure to the saturated vapor pressure of the hydrocarbon at this temperature (maximum value: about critical pressure Pc). The ratio of the gas and liquid of the hydrocarbon should be varied depending upon the concentrations of the alcohol in a raw material and the absolute alcohol in a product and can be controlled by the recycling quanity of the hydrocarbon and the mode of giving heat quantity to a reboiler 6.

The above described conditions in the first distillation column are varied with a solvent to be used, but in general, the following conditions are preferably employed.

| Solvent | Critical Temperature (°C) | Critical Presssure (atm) | Commonly Used Range under Gas-Liquid Coexisting Conditions* | | Ordinary Range of Gas/Liquid Weight Ratio** |
|---|---|---|---|---|---|
| | | | Temp. (°C) | Pressure (atm) | |
| $C_3H_6$ | 92.3 | 45.0 | 20 ~ 92 | 10 ~ 45 | 6 ~ 20 |
| $C_3H_8$ | 96.8 | 42.0 | 20 ~ 96 | 8 ~ 42 | 6 ~ 20 |
| n-$C_4H_{10}$ | 152.0 | 37.5 | 20 ~ 152 | 2 ~ 37 | 6 ~ 20 |
| i-$C_4H_{10}$ | 135.0 | 36.0 | 20 ~ 135 | 3 ~ 36 | 6 ~ 20 |

Note)
* strictly, saturated vapor pressure at the temperature and composition
** For example, when water-containing alcohol ($C_2H_5OH$ 100 kg/h, $H_2O$ 5 kg/h) and propane (2100 kg/h) are fed to the first distillation column, an overhead gas ($C_3H_8$ 2000 kg/h, $H_2O$ 4.9 kg/h) is withdrawn from the overhead part and a bottom liquid ($C_3H_8$ 100 kg/h, $C_2H_5OH$ 100 kg/h, $H_2O$ 0.1 kg/h) is withdrawn from the bottom part, during which gas/liquid weight ratio = 2004.9/200.1 = 10.0.

The overhead gas (consisting of a hydrocarbon gas and water, substantially free from alcohol) of the first distillation column for effecting the pressurized extraction is taken out of a line 3, recompressed by a compressor 5 and guided to a reboiler 6 through a line 26, during which the compression heat generated by the recompression of the overhead gas is preferably used as a heat source of the reboiler 6. This operation can be carried out while maintaining the temperature difference between the overhead and bottom of the first distillation column within at most 10°C, so that the temperature of the overhead gas can be raised to

4

that of the bottom or higher by a slight compression of the overhead gas at the compressor 5, i.e. a slight power and the latent heat of condensation of the overhead gas can sufficiently be utilized as a heat source of the reboiler 6. Accordingly, saving of energy can be done in this way to a larger extent as compared with the prior art distillation method.

When the overhead temperatrue of the first distillation column 2 is T °C, the bottom temperature is generally about (T + 4) ~ (T + 6), so the temperature of the overhead gas (T °C ) is raised to give such at least a temperature difference (about 5 °C ) as to be heat-exchanged with the bottom liquid (about T + 5 °C ) utilizing the temperature raising effect by adiabatic compression at the compressor 5. That is, the compression is carried out by the compressor 5 in such a manner that the gas temperature is raised from T °C to about (T + 10) °C . To this end, the compression ratio (outlet pressure/inlet pressure) is ordinarily adjusted to 1.2 to 1.5. The compressed gas is condensed at (T + 10) °C and all of the latent heat of condensation and a part of the sensible heat thereof are given to the bottom liquid of the first distillation column, whereby the substantial energy required for the distillation can almost be given.

The mixture of the hydrocarbon and water, heat-exchanged at the reboiler 6, is fed through a cooler 7 to a decanter 8, where the water content and hydrocarbon are subjected to separation by gravity-settling. The water is withdrawn through a line 9, and the hydrocarbon substantially free from water is recycled to the first distillation column 2 for pressurized extraction and the second distillation column 25 for stripping through lines 10, 11 and 12, reducing valves 13 and 15, and lines 14 and 16.

The mixture of absolute alcohol substantially free from water and liquid hydrocarbon is withdrawn from a bottom liquid withdrawing line 4 of the first distillation column 2, guided to a heat-exchanger 19 through a line 18 provided with a bottom level controlling valve 17 of the first column 2, heated and further fed through a line 20 to the middle part of the second distillation column 25 functioning as a stripper. This second distillation column is also a pressurized column which gas-liquid contact part consists of a packed column, perforated plate or tray. The pressure of the second distillation column 25 is preferably somaintained that the pressure difference from the first distillation column 2 is substantially zero or as small as possible.

The overhead gas (hydrocarbon gas substantially free from alcohol) of the second distillation column 25 is taken out of a line 21, mixed with the gas of the line 3 of the first distillation column 2 and recompressed by the compressor 5.

The inventors have confirmed that it is more economical to recompress the gases of the lines 3 and 21 by one compressor than separating recompressing and thus the feature of the present invention consists in effecting the operation as in the former case.

As described above, it is confirmed in practicing the present invention that the smaller is the pressure difference between the first distillation column 2 and the second distillation column 25, the smaller is the energy required for compressig and the operation is most economical when the difference is zero. In a preferred embodiment of the present invention, provision of such onditions is carried out.

On the other hand, absolute alcohol substantially containing no hydrocarbon ( at least 99.2 weight % of ethanol) is withdrawn from the bottom of the the second distillation column 25 via a line 22. Since the temperature of the absolute alcohol withdrawn from the line 22 is substantially the same as the boiling point of alcohol under pressure, the absolute alcohol is guided to the heat exchanger 19 via the line 22, where the heat thereof is recovered and utilized for raising the temperature of the fluid of the line 18, and it is withwidrawn as a product. 4 designates a reboiler of the second distillation column 25.

The present invention will now be illustrated in detail by the following examples.

Example 1

The process of the present invention shown in the flow of Fig. 1 is compared with the reference process, before improved as in the present invention, shown in the flow of Fig. 2 (a process comprising compressing separately the gaseous phases leaving the upper parts of the first distillation column and second distillation column by means of different compressors and utilizing respectively the heat thereof as heat sources of the distillation columns; the same numerals in Fig. 2 as in Fig. 1 representing the same apparatus) using propane, propylene, n-butane and i-butane as the hydrocarbon solvent, as to the energy required, installation cost and product alcohol concentration, thus obtaining results as shown in Table 1.

The specification of the main instruments is as follows:

| First Distillation Column: | inner diameter:<br>height: | 100 mm<br>4 m |
|---|---|---|
| | packed column system | |
| Second Distillation Column: | inner diameter:<br>height: | 100 mm<br>4 m |
| | packed column | |

Thus, it is confirmed that the energy required and installation cost can largely be deceased according to the present invention.

Table 1

| Hydrocarbon Solvent | Propane | | | | Propylene | | n-Butane | | i-Butane | |
|---|---|---|---|---|---|---|---|---|---|---|
| RUN No. | 1-1 | 1-2 | 1-3 | 1-4 | 2-1 | 2-2 | 3-1 | 3-2 | 4-1 | 4-2 |
| 1) Process Flow | Fig. 2 | Fig. 1 | Fig. 2 | Fig. 1 | Fig. 2 | Fig. 1 | Fig. 2 | Fig. 1 | Fig. 2 | Fig. 1 |
| 2) Raw Material | | | | | | | | | | |
| Flow Rate (kg/h) | 0.1 | 0.1 | 0.1 | 0.1 | 0.1 | 0.1 | 0.1 | 0.1 | 0.1 | 0.1 |
| Alcohol Concentration (wt %) | 95 | 95 | 95 | 95 | 95 | 95 | 95 | 95 | 95 | 95 |
| 3) First Distillation Column | | | | | | | | | | |
| Pressure (kg/cm²G) | 9 | 9 | 33 | 33 | 9 | 9 | 1 | 1 | 1.5 | 1.5 |
| Temperature (°C) Overhead | 26 | 26 | 83 | 83 | 20 | 20 | 20 | 20 | 17 | 17 |
| Bottom | 30 | 30 | 88 | 88 | 25 | 25 | 24 | 24 | 21 | 21 |
| Solvent Flow Rate (kg/h) | 5 | 5 | 2 | 2 | 5 | 5 | 5 | 5 | 5 | 5 |
| 4) Second Distillation Column | | | | | | | | | | |
| Pressure (kg/cm²G) | 8 | 9 | 8 | 33 | 8 | 8 | 1 | 1 | 1.5 | 1.5 |
| Temperature (°C) Overhead | 25 | 26 | 25 | 83 | 16 | 16 | 20 | 20 | 17 | 17 |
| Bottom | 150 | 150 | 150 | 207 | 150 | 150 | 97 | 97 | 105 | 105 |
| Reflux Ratio | 0.5 | 0.5 | 0.5 | 0.5 | 0.5 | 0.5 | 0.5 | 0.5 | 0.5 | 0.5 |
| Energy Required (kcal/kg alcohol) | 210 | 200 | 195 | 180 | 209 | 183 | 215 | 185 | 214 | 181 |
| Comparison of Installation Cost (-) | 100 | 68 | 100 | 70 | 100 | 72 | 100 | 69 | 100 | 70 |
| Concentration of Product Ethanol (wt %) | 99 or more | ↓ | ↓ | ↓ | ↓ | ↓ | ↓ | ↓ | ↓ | ↓ |
| Recovery of Product Ethanol (wt %) | 99 or more | ↓ | ↓ | ↓ | ↓ | ↓ | ↓ | ↓ | ↓ | ↓ |
| Total Evaluation | △ | ○ | △ | ◎ | △ | ◎ | △ | ◎ | △ | ◎ |

Comparative Example 1

Procedure of Example 1 was repeated except using $CO_2$, $C_2H_6$, $n\text{-}C_6H_{14}$ and benzene as a solvent in the flow of Fig. 1 to obtain results as shown in Table 2.

EP 0 453 569 B1

When using these solvents, the ethanol concentration in the product was not increased and there was not obtained absolute alcohol with a concentration of at least 99.2 % by weight.

Table 2

| | | CO$_2$ | | C$_2$H$_6$ | | n-C$_6$H$_{14}$ | | | Benzene | | |
|---|---|---|---|---|---|---|---|---|---|---|---|
| Solvent RUN No. | | 5-1 | 5-2 | 6-1 | 6-2 | 7-1 | 7-2 | 7-3 | 8-1 | 8-2 | 8-3 |
| 1) Process Flow | | Fig.1 | ↓ | ↓ | ↓ | ↓ | ↓ | ↓ | ↓ | ↓ | ↓ |
| 2) Raw Material | | | | | | | | | | | |
| Flow Rate (kg/h) | | 0.1 | ↓ | ↓ | ↓ | ↓ | ↓ | ↓ | ↓ | ↓ | ↓ |
| Alcohol Concentration (wt %) | | 95 | ↓ | ↓ | ↓ | ↓ | ↓ | ↓ | ↓ | ↓ | ↓ |
| 3) First Distillation Column | | | | | | | | | | | |
| Pressure (kg/cm²G) | | 50 | 50 | 36 | 36 | 1 | 1 | 5 | 1 | 1 | 5 |
| Temperature (°C) Overhead | | 20 | 20 | 20 | 20 | 94 | 94 | 140 | 106 | 106 | 152 |
| Bottom | | 31 | 32 | 33 | 35 | 104 | 105 | 160 | 122 | 124 | 165 |
| Solvent Flow Rate (kg/h) | | 5 | ↓ | ↓ | ↓ | ↓ | ↓ | ↓ | ↓ | ↓ | ↓ |
| Concentration of Product Ethanol (wt %) | | 90 | 91 | 92 | 89 | 95 | 95 | 94 | 94 | 94 | 93 |
| Total Evaluation | | × | × | × | × | × | × | × | × | × | × |

Utility and Possibility on Commercial Scale

In the production of absolute alcohol by separating water content from an aqueous alcohol solution, according to the present invention as described above in detail, absolute alcohol with a purity of at least 99.2 % by weight, capable of satisfying the Government Monopoly in Alcohol Act and JIS Standard, can readily be obtained by carrying out pressurized extractive distillation using propane, propylene or butane as a hydrocarbon solvent, and saving of energy can be accomplished to a greater extent by a heat pump system utilizing the recompression heat of the overhead gas, as compared with the prior art distillation method.

**Claims**

1. A process for the production of absolute alcohol which process comprises feeding a raw material containing ethanol and water as predominant components to the middle part of a first distillation column, feeding a hydrocarbon solvent consisting of a $C_3$ or $C_4$ hydrocarbon or a mixture thereof to the upper part of the first distillation column, maintaining the hydrocarbon at a temperature and pressure such that the liquid and gas phases of the hydrocarbon are simultaneously present in the first distillation column, withdrawing ethanol and liquid hydrocarbon substantially free from water from the lower part of the first distillation column and withdrawing water and hydrocarbon vapour substantially free from ethanol from the upper part of the distillation column, feeding the mixed liquid solution of ethanol and hydrocarbon from the lower part of the first distillation column to a second distillation column where the hydrocarbon solvent is stripped from the ethanol, mixing the gaseous phase from the upper part of the second distillation column with the gaseous phase from the upper part of the first distillation column, compressing the mixture, using the resulting heat of compression as a heat source for the first distillation column, and then cooling and liquidifying the mixture, separating the water, and recycling the hydrocarbon solvent to the upper parts of the first and second distillation columns.

2. A process as claimed in claim 1 wherein the hydrocarbon is propane, propylene, n-butane or iso-butane.

3. A process as claimed in claim 1 or claim 2 wherein the pressure difference between the first distillation column and the second distillation column is kept as low as possible.

4. A process as claimed in claim 3 wherein the pressure difference is substantially zero.

5. A process as claimed in any one of the preceding claims wherein heat contained in the absolute alcohol recovered from the process is used to heat the mixture of ethanol and hydrocarbon fed to the second distillation column.

**Patentansprüche**

1. Verfahren zur Herstellung von absolutem Alkohol, bei dem man
ein aus Ethanol und Wasser als Hauptkomponenten bestehendes Rohmaterial in den Mittelteil einer ersten Destillationssäule einspeist,
ein Kohlenwasserstofflösungsmittel aus einem $C_3$- oder $C_4$-Kohlenwasserstoff oder einem Gemisch derselben in den oberen Teil der ersten Destillationssäule einspeist,
den Kohlenwasserstoff bei einer solchen Temperatur und einem solchen Druck hält, daß in der ersten Destillationssäule sowohl Flüssig- als auch Gasphase des Kohlenwasserstoffs gleichzeitig vorliegen,
Ethanol und flüssigen Kohlenwasserstoff im wesentlichen wasserfrei aus dem unteren Teil der ersten Destillationssäule und Wasser und Kohlenwasserstoffdampf im wesentlichen frei von Ethanol aus dem oberen Teil der ersten Destillationssäule entnimmt,
die gemischte flüssige Lösung aus Ethanol und Kohlenwasserstoff aus dem unteren Teil der ersten Destillationssäule in eine zweite Destillationssäule einspeist, wo das Kohlenwasserstofflösungsmittel vom Ethanol abgezogen wird,
die Gasphase aus dem oberen Teil der zweiten Destillationssäule mit der Gasphase aus dem oberen Teil der ersten Destillationssäule mischt,
die Mischung komprimiert,

die daraus resultierende Kompressionswärme als Warmequelle für die erste Destillationssäule verwendet und

anschließend die Mischung abkühlt und verflüssigt,

das Wasser abtrennt und

das Kohlenwasserstofflösungsmittel zum oberen Teil der ersten und der zweiten Destillationssäule zurückführt.

2. Verfahren gemäß Anspruch 1, bei dem der Kohlenwasserstoff Propan, Propylen, n-Butan oder Isobutan ist.

3. Verfahren gemäß Anspruch 1 oder 2, bei dem die Druckdifferenz zwischen der ersten und der zweiten Destillationssäule so gering wie möglich gehalten wird.

4. Verfahren nach Anspruch 3, bei dem die Druckdifferenz praktisch Null ist.

5. Verfahren gemäß einem der vorstehenden Ansprüche, bei dem die im aus dem Verfahren gewonnenen absoluten Alkohol enthaltene Wärme verwendet wird, um das in die zweite Destillationssäule eingespeiste Gemisch aus Ethanol und Kohlenwasserstoff zu erhitzen.

**Revendications**

1. Procédé pour la fabrication d'alcool absolu, ledit procédé comprenant les étapes consistant à alimenter la partie centrale d'une première colonne de distillation avec un matériau brut contenant de l'éthanol et de l'eau comme composants prédominants, à alimenter la partie supérieure de la première colonne de distillation avec un solvant hydrocarboné constitué par un hydrocarbure en $C_3$ ou $C_4$ ou un mélange de ceux-ci, à maintenir l'hydrocarbure à une température et une pression telles que les phases liquide et gazeuse de l'hydrocarbure sont présentes simultanément dans la première colonne de distillation, à extraire de la partie inférieure de la première colonne de distillation l'éthanol et l'hydrocarbure liquide substantiellement exempts d'eau et à extraire l'eau et la vapeur d'hydrocarbure substantiellement exemptes d'éthanol de la partie supérieure de la colonne de distillation, à alimenter avec la solution liquide de mélange d'éthanol et d'hydrocarbure de la partie inférieure de la première colonne de distillation une seconde colonne de distillation dans laquelle le solvant hydrocarboné est séparé de l'éthanol, à mélanger la phase gazeuse de la partie supérieure de la seconde colonne de distillation avec la phase gazeuse provenant de la partie supérieure de la première colonne de distillation, à comprimer le mélange, à utiliser la chaleur résultant de la compression comme source de chaleur pour la première colonne de distillation, et ensuite à refroidir et à liquéfier le mélange, à séparer l'eau, et à recycler le solvant hydrocarboné vers les parties supérieures des première et seconde colonnes de distillation.

2. Procédé selon la revendication 1, dans lequel l'hydrocarbure est du propane, du propylène, du n-butane ou de l'iso-butane.

3. Procédé selon la revendication 1 ou 2, dans lequel la différence de pression entre la première colonne de distillation et la seconde colonne de distillation est maintenue aussi basse que possible.

4. Procédé selon la revendication 3, dans lequel la différence de pression est substantiellement nulle.

5. Procédé selon l'une quelconque des revendications précédentes, dans lequel la chaleur contenue dans l'alcool absolu récupéré selon le procédé est utilisée pour chauffer le mélange d'éthanol et d'hydrocarbure alimenté dans la seconde colonne de distillation.

# FIG. 1

EP 0 453 569 B1

# FIG. 2

EP 0 453 569 B1